(19) Europäisches Patentamt
European Patent Office
Office européen des brevets

(11) Numéro de publication: **0 114 850**
**B1**

(12) **FASCICULE DE BREVET EUROPÉEN**

(45) Date de publication du fascicule du brevet: **29.06.88**

(21) Numéro de dépôt: **83902270.4**

(22) Date de dépôt: **26.07.83**

(86) Numéro de dépôt international: **PCT/FR 83/00158**

(87) Numéro de publication internationale: **WO 84/00546 (16.02.84 Gazette 84/05)**

(51) Int. Cl.⁴: **C 07 D 401/12**, C 07 D 413/12, C 07 D 405/14, C 07 D 413/14, C 07 D 401/14, A 61 K 31/445 // C07D405/06, C07D401/06

(54) **NOUVEAUX DERIVES SUBSTITUES DU 2,5-DIAMINO 1,4-DIAZOLE, LEURS PROCEDES DE PREPARATION ET LES COMPOSITIONS PHARMACEUTIQUES EN RENFERMANT.**

(30) Priorité: **26.07.82 FR 8213010**

(43) Date de publication de la demande: **08.08.84 Bulletin 84/32**

(45) Mention de la délivrance du brevet: **29.06.88 Bulletin 88/26**

(84) Etats contractants désignés: **AT BE CH DE FR GB LI LU NL SE**

(56) Documents cité:
**FR-A-2 105 223**
**FR-A-2 183 683**
**FR-A-2 213 059**
**FR-M-7 787**
**US-A-3 238 215**
**US-A-3 966 748**
**US-A-4 140 781**

(73) Titulaire: **BOUCHARA S.A., 47, rue de Bretagne, F-75003 Paris (FR)**

(72) Inventeur: **CORNU, Pierre, Jean, 100, avenue Kléber, F-75116 Paris (FR)**
Inventeur: **PERRIN, Claude, 5, rue de l'Avenir, F-91400 Orsay (FR)**
Inventeur: **DUMAITRE, Bernard, 24, rue Chemin-Vert, F-93000 Bobigny (FR)**
Inventeur: **STREICHENBERGER, Gilles, 30, bld du Château, F-92200 Neuilly- sur- Seine (FR)**

(74) Mandataire: **Burtin, Jean- François, Laboratoires Bouchara 8, rue Pastourelle, F-75003 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 114 850 B1

LIBER, STOCKHOLM 1988

**Description**

L'invention concerne de nouveaux dérivés hétérocycliques azotés, leurs procédés de préparation et les compositions pharmaceutiques en renfermant.

Elle a plus particulièrement pour objet de nouveaux dérivés du 2,5-diamino 1,4-diazole dont les groupes aminés peuvent être substitués.

Elle a spécifiquement pour objet les 2-amino 5-[(aralcoyl-pipéridino alcoyl) amino] 1,4-diazoles de formule générale I

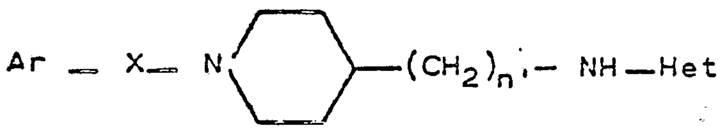

(I)

dans laquelle Ar est un radical aryle ou hétéroaryle, mono - ou bicyclique tel que défini plus loin,

X est une chaîne alcoylène éventuellement substituée, choisie dans le groupe constitué

$(CH_2)_{n'}CHOH$ - $(CH_2)_{n_1}$ - CO $(CH_2)_{n_1}$ - ou C - $(CH_2)_{n_1}$
ǁ
K

n est égal à 1, 2, 3 ou 4
$n_1$ est égal à 1, 2 ou 3
n' est égal à 0, 1 ou 2

K est un groupe 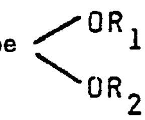

ou une chaîne alcoylènedioxy

Het est un radical choisi dans le groupe constitué par les composés de formule (A)

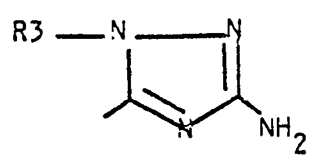

(A)

dans laquelle $R_3$ est de l'hydrogène, un radical alcoyle en $C_1$-$C_6$, aryle ou aralcoyle inférieur en $C_1$-$C_6$ pour la partie alcoyle, le terme aryle étant défini plus loin et les composés de formule B

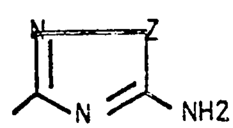

(B)

dans laquelle Z est un radical NH, N-R3 ou de l'oxygène (dans lequel R3 est un radical alcoyle en $C_1$-$C_6$, aryle ou aralcoyle inférieur en $C_1$-$C_6$ pour la partie aryle, la terme aryle étant défini plus loin.

L'invention concerne également les sels d'addition des composés de formule générale I avec un acide minéral ou organique, de préférence thérapeutiquement compatible.

L'invention concerne aussi les isomères optiques des composés de formule générale I. La molécule comporte au moins un carbone asymétrique et de ce fait on peut séparer les isomères droit ou gauche.

En outre, lorsque X est une chaîne -[CHOH-(CH2)]$_{n_1}$ il apparaît un nouveau centre d'asymétrie et il est possible d'isoler les diastéréo isomères en résultant, qui à leur tour pourront être dédoublés en leurs isomères optiques.

Enfin, lorsque Ar est un radical bicyclique partiellement saturé, il existe un atome de carbone asymétrique supplémentaire, donnant naissance à une nouvelle possibilité de dédoublement. La molécule peut donc s'écrire dans ce cas de la manière suivante:

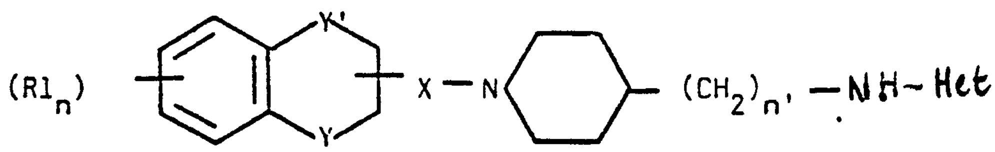

Dans cette structure Y et Y' sont, indépendamment l'un de l'autre, un radical - $CH_2$-, -O, -S- ou NH-.

Lorsque le substituant Z représente de l'oxygène, la structure hétérocyclique est celle d'un 2,5-diamino-3-oxa 1,4-diazole. Lorsque le substituant Z représente le radical imino -NH- la structure hétérocyclique est celle d'un 2,5-diamino 1,3,4-triazole. Celui-ci peut exister sous les deux formes imino triazole tautomères.

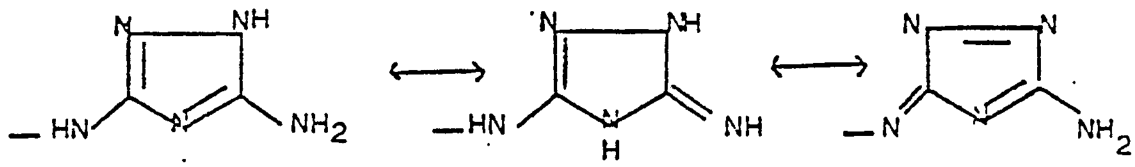

le groupe Ar a la signification suivante: Ar est un radical aryle mono-cyclique choisi dans le groupe constitué par la pyridine, l'oxazine, la pyrazine et un radical phényle de formule générale

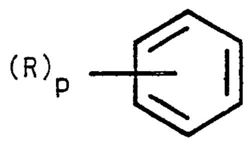

dans laquelle R est de l'hydrogène, un radical alcoyle en $C_1$-$C_6$, un radical alcoxy en $C_1$-$C_6$, un halogène, un trifluorométhyle, un radical sulfonamido, un radical trifluorométhoxy, un radical cyano, un radical carboxy, un radical carbalcoxy, un radical carboxamido, un groupe nitro ou un groupe alcoylène-dioxy en $C_1$-$C_6$ et p est un nombre entier variant de 1 à 3;

ou un radical aromatique bicyclique non saturé ou partiellement saturé choisi dans le groupe constitué parle tétrahydronaphtyle, le naphtyle-1, le naphtyle-2, le benzodioxanyle, le benzodioxényle, le quinoléinyle, le thiachromanyle, l'indolyle-2 et l'indolyle-3.

Le substituant X est une chaîne alcoylène ayant de 1 à 4 atomes de carbone en chaîne droite ou ramifiée, une chaîne hydroxy alcoylène ayant 2, 3 ou 4 atomes de carbone ou une chaîne oxoalcoylène ayant 2, 3 ou 4 atomes de carbone ou une chaîne oxoalcoylène cétalisée ayant 2, 3 ou 4 atomes de carbone.

La nature et la longueur des chaînes alcoylène en fonction des substituants n, $n_1$ et n' jouent un rôle important et conditionnent l'intensité ou la durée des effets pharmacologiques des composés de formule générale I.

Parmi les composés de formule générale I, on distinguera plus particulièrement 5 sous-groupes actuellement préférés:

a) <u>les (phénylalcoyl) pipéridines de formule générale $I_A$</u>

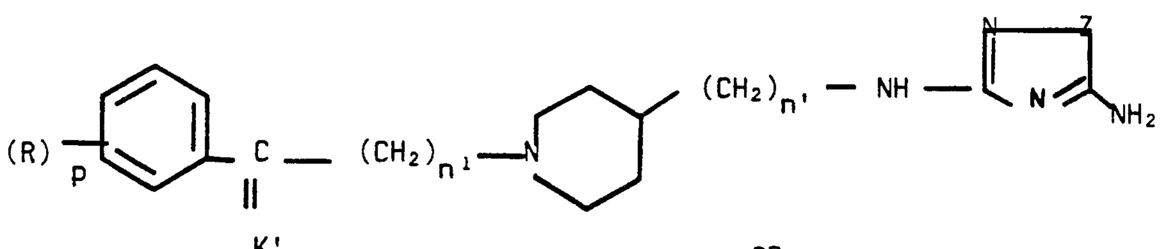

$(I_A)$

dans laquelle K' représente deux hydrogènes, un groupe 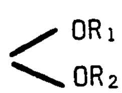

FIG00/10

dans lequel $R_1$ ou $R_2$ représentent chacun un radical alcoyle en $C_1$-$C_6$ ou forment ensemble une chaîne alcoylène ayant de 2 à 4 atomes de carbone, la paire

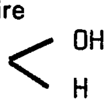

ou l'oxygène d'une fonction cétone

$n_1$ et n' sont définis comme précédemment

et Z représente un groupe NH, $NR_3$ ou de l'oxygène ($R_3$ étant défini comme précédemment) sous forme racémique ou optiquement-active.

b) <u>les (phénylalcoyl) pipéridines de formule générale $I_B$</u>

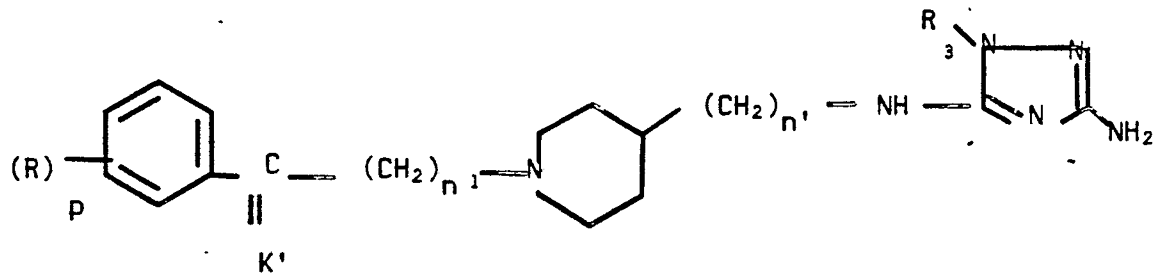

($I_B$)

dans laquelle les substituants R, $R_3$, K', p, $n_1$ et n' sont définis comme précédemment sous forme racémique ou optiquement-active.

c) les (benzo 1,4-dioxanyl) alcoyl pipéridines de formule générale $I_C$

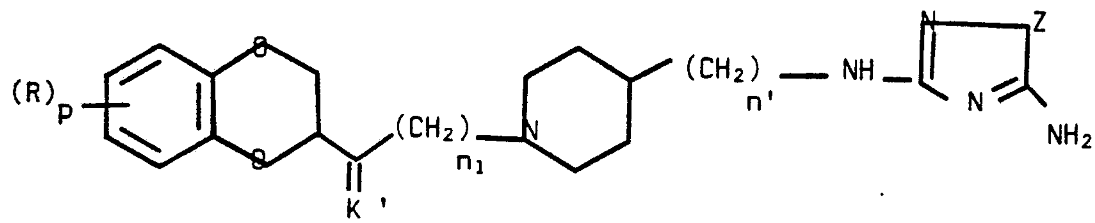

($I_C$)

dans laquelle R, K', Z, p, n' et $n_1$ ont les définitions antérieures, sous forme racémique ou optiquement-active.

d) les (benzo 1,4-dioxanyl) alcoyl pipéridines de formule générale $I_D$

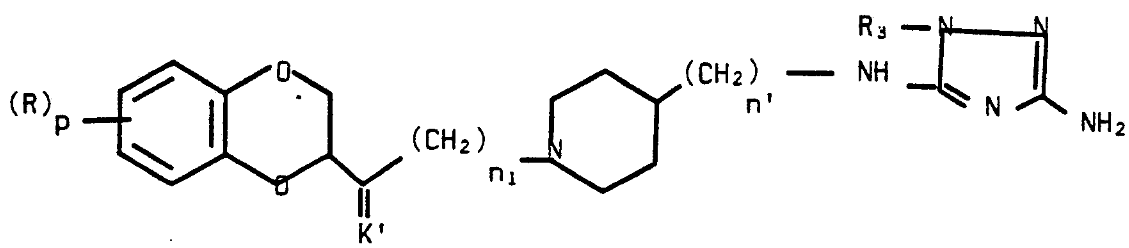

($I_D$)

dans laquelle les substituants R, K', $R_3$, $n_1$, n' et p sont définis comme précédemment sous forme racémique ou optiquement-active.

e) les indolyl (2 ou 3) alcoyl pipéridines de formule générale $I_E$

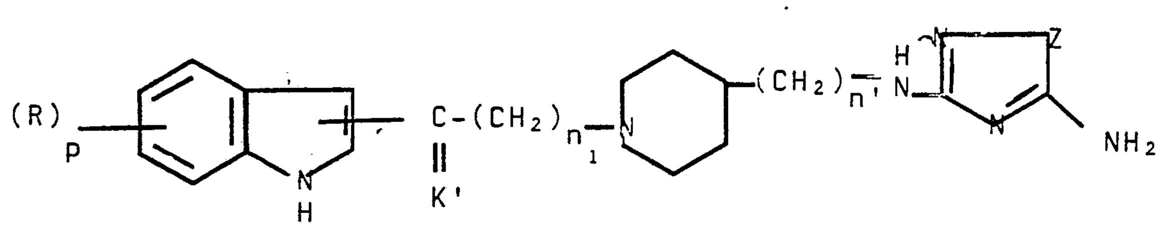

($I_E$)

dans laquelle les substituants R, K', Z, p, $n_1$ et n' sont définis comme précédemment.

Pour autant que l'invention soit concernée, un radical alcoyle inférieur est un radical hydrocarboné ayant de 1 à 6 atomes de carbone en chaîne droite ou ramifiée, comme par exemple le méthyle, l'éthyle, l'isopropyle, le secbutyle, le terbutyle, le pentyle, le néopentyle et le n-hexyle.

Un radical alcoxy inférieur a de 1 à 6 atomes de carbone dans la chaîne alcoyle qui peut être droite ou ramifiée comme un méthoxy, un éthoxy, un isopropoxy, un terbutoxy ou un n-pentyloxy.

On désigne par le vocable "Halogène" un atome de chlore, de brome, d'iode ou de fluor. Le substituant préféré est le fluor.

Le terme aryle désigne un radical aromatique monocyclique, homo ou hétérocyclique, éventuellement substitué par 1, 2 ou 3 substituants choisis dans le groupe constitué par un alcoyle inférieur, un halogène, un alcoxy inférieur, un alcoylène-dioxy et trifluorométhyle.

Le terme aralcoyle désigne un radical aromatique défini comme ci-dessus, substitué par une chaîne alcoyle ayant de 1 à 6 atomes de carbone, droite ou ramifiée.

Parmi les sels d'addition des composés de formule générale I, on pourra citer plus particulièrement les chlorhydrates, bromhydrates, sulfates, nitrates, phosphates, thiosulfates, les formiates, acétates, maléates,

fumarates, benzoates, dichloro 2,6-benzoates, citrates, tartrates, (méthoxy salicylates), 3,4,5-triméthoxy benzoates, les vanillates, les O-carbéthoxy syringoates, les naphtoates, les benzène sulfonates, les méthane sulfonates, les iséthionate les nicotinates, les isonicotinates, les embonates et les glucose-phosphates.

Les sels d'acides qui ne sont pas utilisables en thérapeutique comme les Iodates, perchlorates, thiocyanates, ferrocyanures, oxalates, flavianates ou Reineckates peuvent servir de moyens d'identification, de purification ou d'isolement.

Les composés, selon l'invention, se distinguent par leurs propriétés pharmacologiques intéressantes et notamment par leurs propriétés anti-hypertensives associées à une action faiblement sédative sur le système nerveux central. Du fait de leur haut niveau d'activité, les composés de formule générale I ou leurs sels d'addition trouvent un emploi en thérapeutique humaine ou animale comme principes actifs de médicaments destinés à combattre ou à réduire les effets de la maladie hypertensive.

A ces fins ils sont utilisés sous forme de compositions pharmaceutiques destinées à l'administration par voie parentérale, buccale, rectale ou sublinguale.

Les compositions pharmaceutiques renferment, comme principe actif, au moins un composé de formule générale I ou un de ses sels d'addition avec un acide minéral ou organique, en association ou en mélange avec un excipient ou un véhicule inerte pharmaceutiquement - acceptable.

On pourra citer comme formes d'administration préférées, les comprimés nus ou enrobés, les capsules, les gélules, les dragées, les comprimés à noyaux multiples, les gouttes, les solutions ou suspensions buvables; les solutés ou suspensions injectables répartis en ampoules, en flacons multidoses ou en seringues auto-injectables; les suppositoires; les comprimés sublinguaux.

Les compositions pharmaceutiques, selon l'invention peuvent, en outre, renfermer un ou d'autres principes actifs d'action similaire, complémentaire ou synergique. On pourra ainsi ajouter un diurétique du type thiazidique ou du type triaminoptéridine; ou un agent beta-bloqueur comme le propranolol, le pindolol ou l'aténolol.

La posologie journalière peut varier entre des limites larges, fonction de l'indication thérapeutique, de la voie d'administration, de l'âge du malade et de l'ancienneté de la maladie hypertensive. En règle générale, chez l'adulte, la posologie s'échelonne entre 0,1 et 50 mg par prise et entre 0,1 et 150 mg par jour.

D'une manière préférée les compositions pharmaceutiques selon l'invention, renferment entre 0,1 et 25 mg par prise unitaire, de principe actif de formule générale I ou un de ses sels.

L'invention comprend aussi un procédé pour préparer les composés de formule générale I caractérisé en ce que l'on soumet une N-cyano isothio- ou isourée de formule générale II

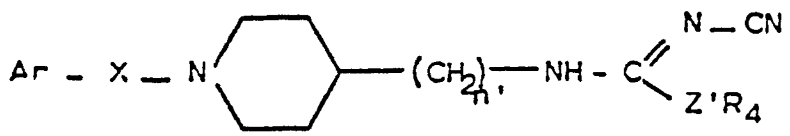

(II)

dans laquelle Ar, X et n' ont les définitions fournies antérieurement

Z' est de l'oxygène ou du soufre

et $R_4$ est un radical alcoyle en $C_1$-$C_6$

à l'action d'un dérivé aminé de formule générale III

$HZ-NH_2$ (III)

dans laquelle Z est défini comme précédemment pour former - lorsque Z est de l'oxygène - le 2-amino 3-oxa 1,4-diazole de formule générale (I')

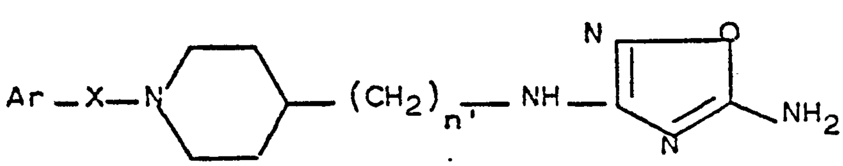

(I')

- lorsque Z est le radical imino NH- un 2-amino 1,3,4-triazole de formule I''

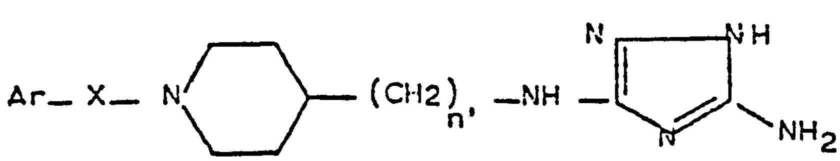

(I")

et lorsque Z est un radical $NR_3$ un mélange de 2-amino 1,3,4-triazoles substitués de formule

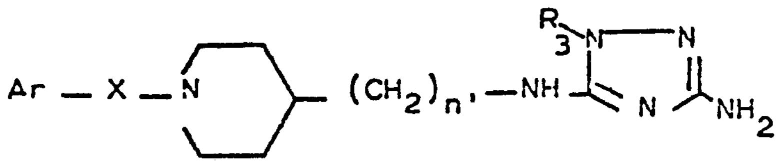

et

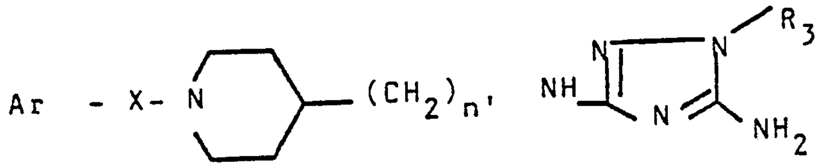

que l'on sépare par des moyens physiques.

Lorsque X est une chaîne $\underset{\underset{K}{\|}}{C}(CH_2)_{n_1}$ - dans laquelle K représente un groupe 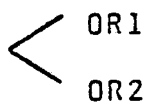

(dans lequel $R_1$ et $R_2$ sont définis comme précédemment), il est possible d'hydrolyser le cétal obtenu par un acide minéral ou organique, ou par échange de fonction avec un acide carbonylé, pour obtenir un composé carbonylé de formule générale IV

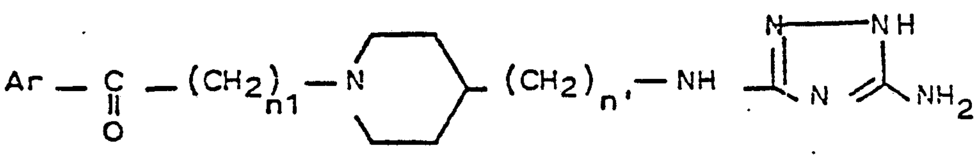 (IV)

dans laquelle Ar, $n_1$, n' et Het sont définis comme précédemment. Les composés de formule II peuvent être obtenus par les procédés décrits dans les demandes EP-83 901 601 et EP-83 901 083.

Le composé carbonylé de formule générale (IV) peut également être réduit en dérivé hydroxylé par action d'un hydrure mixte de métal alcalin comme un borohydrure de métal alcalin ou un aluminohydrure de métal alcalin, par échange de fonction avec un alcool secondaire en présence d'isopropylate d'aluminium dans les conditions de la réaction de Meerwein ou par action d'un métal alcalin comme le sodium en présence d'un alcanol inférieur - pour former un composé hydroxyalcoylé de formule générale V

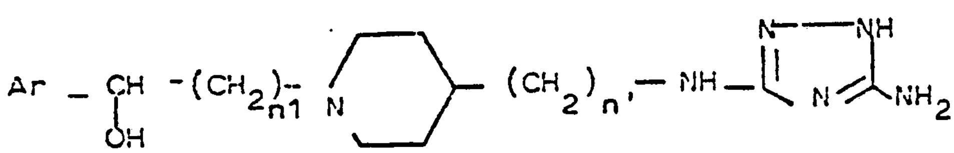 (V)

Le composé corbonylé peut également être réduit en dérivé alcoylé par l'hydrazine dans les conditions de la réaction de Wolff-Kishner ou par le zinc en milieu chlorhydrique dans les conditions de la réaction de Clemmensen pour obtenir un composé alcoylé.

Le dédoublement des composés de formule générale I peut être effectué par salification à l'aide d'un acide optiquement-actif comme l'acide d-tartrique, l'acide d-dibenzoyl-tartrique, l'acide d-diethyltartramique, l'acide pimarique, l'acide abietique, l'acide d-camphosulfonique, l'acide naphtalène dioxyphosphonique optiquement actif ou l'acide glucose 6- ou 1-phosphorique.

Les cyano isourées ou isothiourées de départ répondant à la formule générale II sont préparées selon un procédé qui consiste en ce que l'on fait réagir une 4-ominopipéridine de formule générale VI

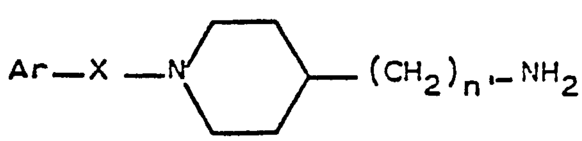 VI

dans laquelle les substituants $A_r$, X et n' sont définis comme précédemment avec un réactif de cyano imination choisi dans le groupe constitué parmi les cyanoimino isodithiocarbonates d'alcoyle de formule générale VII

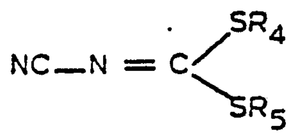

VII

dans laquelle $R_4$ et $R_5$ sont des radicaux alcoyle inférieur, et les cyanoimino isothiocarbonates mixtes d'alcoyle de formule générale VIII

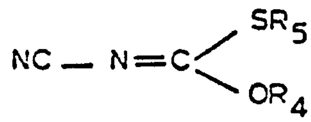

VIII

dans laquelle $R_5$ et $R_4$ sont définis comme précédemment paur former l'isathiourée ou l'isourée de formule générale II

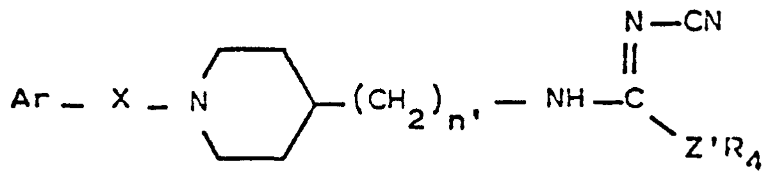

(II)

dans laquelle les substituants $A_r$, $R_4$, et n' sont définis comme précédemment et Z' est de l'oxygène ou du soufre.

Les exemples suivants illustrent l'invention sans toutefois la limiter en aucune façon.

**Exemple 1**

1-(1,4-benzodioxanne 2-yl méthyl) 4-(2-amino S-triazol-5 yl aminométhyl) pipéridine

On porte 4 heures à l'ébullition un mélange de 7,2 g de 1-(1,4-benzodioxanne 2-yl méthyl) 4-(N-cyano S-méthylisothiouréîdométhyl) pipéridine, de 4,5 ml d'hydrate d'hydrazine et 100 ml d'éthanol.

La solution est ensuite concentrée à sec pour donner une pâte qui, traitée à l'acétonitrile, donne des cristaux incolores qui sont séparés par filtration, lavés et séchés.

On obtient ainsi 5 g du produit cherché qui est recristallisé dans l'acétate d'éthyle. Son point de fusion est Fi = 157-8°.

**Exemple 2**

1-(1,4-benzodioxanne 2-yl méthyl) 4-(2-amino S-triazol-5 yl amino) pipéridine

On porte 4 heures à l'ébullition un mélenge de 4 g de 1-(1,4-benzodioxanne 2-yl méthyl) (N-cyano S-méthyl isothiouréîdo) pipéridine et 4 ml d'hydrate d'hydrozine dans 100 ml d'éthanol.

On concentre à sec et la pâte obtenue est reprise à l'acétonitrile ce qui donne 3,2 g de cristaux blancs.

On purifie par recristallisation dans l'isopropanol.

On obtient ainsi la 1-(1,4-benzodioxanne 2-yl méthyl) 4-(2-amino S-triazol-5 yl amino) pipéridine cherchée fondant à : Fi = 215-16°.

**Exemple 3**

1-(1,4-benzodioxanne 2-yl méthyl) 4-(2-amino-1,3,5 oxadiazol-4 yl amino) pipéridine

On porte 4 heures à l'ébullition un mélange de 7 g de 1-(1,4-benzodioxanne 2-yl methyl) 4-(N-cyano S-méthylisothiouréîdo) pipéridine, 4,2 g de chlorhydrate d'hydroxylamine, 5,2 g de bicarbonate de sodium et 20 ml d'éthanol.

La solution est ensuite concentrée à sec ce qui permet d'isoler un solide vitreux qui est repris à l'acétonitrile. Il y a d'abord dissolution, puis cristallisation.

Les cristaux sont filtrés, lavés à l'acétonitrilé et séchés.

On obtient 2,9 g du produit cherché qui présente un double point de fusion, 1ère fusion à 141-2° puis solidification et fusion à 167-8°.

**Exemple 4**

1-(1,4-benzodioxanne-2 yl éthyl) 4-(2-amino S-triazol-5 yl aminométhyl) pipéridine.

On porte 4 heures à l'ebullition, un mélange de 7,5 g de 1-(1,4-benzodioxanne-2 yl éthyl) 4-(N-cyano S-méthylisothiouréîdométhyl) pipéridine, de 4,5 ml d'hydrate d-hydrazine dans 100 ml d'éthanol.

Après concentration à sec, on obtient une pâte qui cristallise por trituration avec l'acétonitrile.

Les cristaux sont filtrés, lavés à l'acétonitrile et séchés.

On purifie par recristallisation dans l'isopropanol ce qui perm' l'obtenir 5 g du produit cherché fondant à : Fi = 148°.

**Exemple 5**

1-(1,4-benzodioxanne-2 yl éthyl) 4-(2-amino-1,3,5 oxadiazol-4 yl aminométhyl) pipéridine

On porte 20 heures à l'ébullition, un mélange de 7,5 g de 1-(1,4-benzodioxanne-2 yl éthyl) 4-(N-cyano S-méthylisothiauréîdométhyl) pipéridine, 2,1 g de chlorhydrate d'hydroxylamine et de 5,1 g de bicarboncte de sodium dans 100 ml d'éthanol.

Après filtration, on concentre à sec pour obtenir une huile demi-solide que l'on dissout à nouveau dans 50 ml d'isapropanol à chaud.

Il y a cristallisation par refroidissement peu après et les cristaux sont filtrés et séchés.

Après purification par recristallisation dans l'éthanol, on obtient 2,6 g du produit cherché qui cristallise avec 1/2 molécule d'eau. Il présente un double point de fusion Fi = d'abord 75° puis solidification et fusion à 128°.

Le produit de départ 1-(1,4-benzodioxanne-2 yl éthyl) 4-(N-cyano S-méthylisothiouréîdométhyl) pipéridine est obtenu de la façon suivante:

On porte 4 heures à l'ébullition un mélange de 73 g de 1-(1,4-benzodioxanne-2 yl éthyl) 4-aminométhyl pipéridine et 38,6 g de N-cyanoimino dithiocarbonate de diméthyle dans 500 ml d'éthanol.

Le produit qui cristallise au refroidissement est filtré, lavé à l'éthanol et séché.

On obtient ainsi 85 g du produit cherché sous farme de cristaux blancs présentant un double point de fusion d'abord 145° puis solidification et fusion à 155°.

**Exemple 6**

1-[(6-méthyl-1,4-benzodioxanne)-2 yl méthyl] 4-(2-amino S-triazol-5 yl aminométhyl) pipéridine

**Stade A**

1-[(6-méthyl 1,4-benzodioxanne)-2 yl méthyl] 4-(N-cyano S-méthylisothiouréîdométhyl) pipéridine

On chauffe 4 heures à l'ébullition un mélange de 58,4 g de 1-[(6-méthyl 1,4-benzodioxanne)-2 yl méthyl] 4-aminométhyl - pipéridine et 31 g de N-cyanoimino isodithiocarbonate de diméthyle dans 400 ml d'éthanol.

Le produit qui cristallise par refroidissement est filtré, lavé à l'éthanol et séché. Il se présente sous farme de cristaux blancs fondant à : Fi = 145°.

**Stade B**

On chauffe 4 heures au reflux 7,5 g de 1-[(6-méthyl 1,4-benzodioxanne)-2 yl méthyl]4-(N-cyano S-méthylisothiouréîdo) pipéridine avec 4,5 ml d'hydrate d'hydrazine dans 140 ml d'éthanol.

Après concentration à sec, on obtient une pâte qui, reprise à l'acétonitrile, cristallise.

On obtient ainsi 6,5 g de cristaux blancs qui peuvent être purifiés par recristallisation dans l'isopropanol.

La 1-[(6-méthyl 1,4-benzodioxanne)-2 yl méthyl] 4-(2-amino S-triazol-5 yl aminométhyl) pipéridine se

présente sous forme de cristaux pratiquement incolores fondant à Fi = 138°.

**Exemple 7**

1-[(6-méthyl 1,4-benzodioxanne)-2 yl méthyl] 4-(2-amino 1,3,5-oxadiazol-4 yl amino méthyl) pipéridine

On chauffe 20 heures à l'ébullition un mélange de 7,5 g de 1-[(6-méthyl 1,4-benzodioxanne-2 yl méthyl] 4-(N-cyano S-méthylisothiouréîdométhyl) pipéridine, de 4,2 g de chlorhydrate d'hydroxylamine et de 10,2 g de bicarbonate de sodium dans 100 ml d'éthanol.

Après filtration, on concentre à sec la solution éthanolique et reprend par l'eau le résidu sec puis l'extrait au chloroforme. On sépare la phase organique.

Après séchage et concentration à sec, on obtient 5,7 g d'un produit qui est recristallisé dans l'isopropanol. Il fond à: Fi = 146°.

**Exemple 8**

1-(1,4-benzodioxonne-2 yl méthyl) 4-(2-amino 1,3,5-oxadiazol-4 yl aminaméthyl) pipéridine

On porte 4 heures à l'ébullition, un mélange de 7,2 g de 1-(1,4-benzodioxanne-2 yl méthyl) 4-(N-cyano S-méthylisothiouréîdométhyl) pipéridine, de 2,1 g de chlorhydrate d'hydroxylamine, de 5,1 g de bicarbanate de sodium dans 100 ml d'éthanol.

Après filtration, on concentre à sec ce qui permet d'obtenir un produit amorphe semi-solide incristallisable.

Ce produit est purifié par passage sur une colonne de silice H. Merck avec le mélange chloroforme - isopropylamine 9 : 1 comme éluant.

Le produit obtenu par élution et évaporation du solvant cristallise alors par traitement avec le minimum d'acétonitrile.

On obtient aussi 3,8 g du produit cherché sous forme de cristaux blancs fondant à: PFi = 130-131°.

**Exemple 9**

1-[(1,4-benzodioxanne-2 yl (hydroxy-2 éthyl)] 4-(2-amino 1,3,5-oxadiazol-4 yl aminométhyl) pipéridine

**Stade A**

1-[1,4-benzodioxanne-2 yl (hydroxy-2 éthyl)]4-(N-cyano S-méthylisothiouréîdométhyl) pipéridine

On chauffe 4 heures à l'ébullition, un mélange de 58 g de 1-[1,4-benzodioxanne-2 yl (hydroxy-2 éthyl)] 4-aminométhyl pipéridino et de 29 g de N-cyanoimino dithiocarbonate de diméthyle dans 400 ml d'éthanol.

La solution est ensuite concentrée à sec et reprise par l'éther isopropylique, ce qui permet d'obtenir des cristaux qui sont séparés par filtration, lavés à l'éther isopropylique et séchés.

Par recristallisation dans l'isopropanol, on obtient 58 g du produit cherché fondant à : Fi = 128°.

**Stade B**

On porte 20 heures à l'ébullition, un mélange de 7,8 g de 1-[1,4-benzodioxanne-2 yl (hydroxy-2 éthyl)] 4-(N-cyano S-méthylisothiouréîdométhyl) pipéridine, de 2,1 g de chlorhydrate d'hydroxylamine et de 5,1 g de bicarbonate de sodium dans 100 ml d'éthanol.

Après filtration, on concentre à sec le mélange réactionnel et on obtient un solide poteux qui est repris à chaud par de l'isopropanol.

Après 24 heures, il y a déposition d'un solide qui est séparé par filtration, lavé à l'éther isopropylique et recristallisé dans le minimum d'éthanol.

On obtient ainsi 1,7 g de produit cherché sous forme de cristaux qui cristallisent avec 1/2 molécule d'eau. Il fond à Fi = 148-150°.

**Exemple 10**

1-[1,4-benzodioxanne-2 yl (hydroxy-2 éthyl)] 4-(2-amino S-triazol-5 yl aminométhyl) pipéridine.

On porte 4 heures à l'ébullition, un mélange de 7,8 g de 1-[1,4-benzodioxanne-2 yl (hydroxy-2 éthyl)]4-(N-cyano S-méthylisothiouréîdométhyl) pipéridine obtenue à l'ex. 8 stade A et da 5 ml d'hydrate d'hydrazine dans 100 ml d'éthanol.

Après concentration à sec, on obtient une pâte qui se solidifie par traitement à l'acétonitrile.

Le produit est filtré, lavé à l'éther isopropylique, séché et recristallisé dans l'isopropanol.

On obtient ainsi 2 g de produit cherché sous forme de cristaux de PFi = 164°.

**Exemple 11**

1-(1,4-benzodioxanne-2 yl méthyl) 4-(2-amino 3-méthyl S-triazol-5 yl aminométhyl) pipéridine et
1-(1,4-benzodioxanne-2 yl méthyl) 4-(2-amino 4-méthyl S-triazol-5 yl aminométhyl) pipéridine

On porte 18 heures à l'ébullition 3,6 g de (1,4-benzodioxanne-2 yl méthyl) 4-(N-cyano S-méthylisothiouréîdométhyl) pipéridine 8 ml de méthylhydrozine et 50 ml d'éthanol.

Après concentration à sec, on obtient une huile qui présente 2 taches en chromatographie couche mince dans le système chloroforme isopropylamine 9-1.

Les deux produits sont séparés sur une colonne de silice H. Merck, avec un mélange chloroforme -isopropylamine 9-1.

On obtient ainsi 1,4 g du produit passant en tête sous forme d'huile qui cristallise par traitament à l'éther isopropylique.

Ce produit correspond au dérivé 3-méthylé. Il fond à : Fi = 129 - 130°.

En continuant l'élution, on sépare 0,7 g d'huile cristallisant par traitement à l'éther isopropylique, correspondant au dérivé 4-méthylé. Ce produit fond à: Fi = 139-40°.

Si on utilise à la place de la méthylhydrazine la phénylhydrazine, on obtient de la même façon un mélange de dérivé 3-phénylé et de dérivé 4-phénylé que l'on sépare par chromatographie sur silice.

Si on utilise de la même manière la p. méthoxyphényl hydrazine on obtient un mélange de dérivé 3-(4-méthoxyphényl) et de dérivé 4-(4-méthoxyphényl) que l'on sépare par chromatographie sur colonne de silice.

Si l'on utilise l'isopropylhydrazine on obtient de la même façon un mélange de dérivé 3-isopropylé et de dérivé 4-isopropylé que l'on sépare par chramatographie sur colonne de silice.

**Exemple 12**

1-(4-phényl 4-oxo)-1 butyl 4-(2-amino S-triozol-5 yl aminométhyl) pipéridine

**Stade A**

[(4-phényl 4,4-éthylènedioxy)-1 butyl-1](N-cyano S-méthylisothiouréîdométhyl)-4 pipéridine

On chauffe 4 heures à reflux une solution de 82,3 g de 1-[(4-phényl 4,4-éthylènedioxy)-1 butyl]4-aminométhyl pipéridine et 39,5 g de N-cyanoimino dithiacarbonate de diméthyle dans 400 ml d'éthanol.

On concentre ensuite à sec et l'huile obtenue est reprise par l'éther isopropylique.

On obtient ainsi 105 g du produit cherché sous forme de cristaux blancs de PFi = 136°.

**Stade B**

On chauffe 18 heures au reflux 8 g de 1-[(4-phenyl 4-amino éthylènedioxy)-1 butyl] 4-[N-cyano S-méthylisotiouréîdométhyl-4] pipéridine avec 4,5 ml d'hydrate d'hydrozine dans 100 ml d'éthanol.

Après concentration à sec, on obtient une huile vitreuse que l'on fait cristalliser par dissolution dans l'acétate d'éthyle et refroidissement.

On obtient ainsi 63 g de produit fondant à 134°.

On hydrolyse l'acétal par dissolution dans 150 ml d'acide chlorhydrique N et un repos de 18 heures à la température ambiante.

On amène ensuite à pH = 10 avec une solution de saude ce qui provoque une cristalisation.

Après filtration, lavage à l'eau et séchage on obtient 5,2 g de produit qui est racristallisé dans l'alcool isopropylique. Le produit pur fond à: Fi = 154°.

**Exemple 13**

1[(4-phényl 4-oxo)-1 butyl] 4 (2-amino 1,3,5 oxadiazol-4 yl aminaméthyl) pipéridine

On chauffe 18 heures au reflux un mélange de 16,1 g 1-[(4-phényl 4,4-éthylènedioxy)-1 butyl] 4-(N-cyano S-méthylisothiouréîdométhyl) pipéridine, de 8,4 g de bicarbonate de sodium et de 7,2 g de chlorhydrate d'hydroxylamine dans 200 ml d'éthanol.

Après filtration, on concentre à sec et obtient un produit semi-solide vitreux que l'on fait cristalliser par traitement à l'acétate d'éthyle à chaud.

Après filtration, lavage et séchage des cristaux, on obtient 6,1 g de produit blanc fondant à: Fi = 126°.

On hydrolyse le cétal par dissolution dans 150 ml d'acide chlorhydrique normal et un repos de 18 heures à la température ambiante.

On rend le milieu basique par une solution de soude ce qui danne une huile qui cristallise par trituratian.

Après filtration, lavage à l'eau et séchage, on obtient 5 g de produit qui est recristallisé dans l'isopropanol. Fi = 80-2°.

**Exemple 14**

1-[(-4-p. fluorophényl 4-oxo)-1 butyl]4-[2-amino S-triazol-5 yl aminométhyl] pipéridine

On porte 16 heures à l'ébullition un mélange de 33,6 g de[(4 p.-fluorophényl 4,4-éthylènedioxy)-1 butyl]-1 (N-cyano S-méthylisothiouréîdométhyl)-4 pipéridine 15 ml d'hydrate d'hydrazine et 400 ml d'éthanol.

Après concentration à sec, on obtient une huile épaisse qui cristallise par traitement à l'acétate d'éthyle.

On obtient ainsi 27,5 g de cristaux de PFi = 145°.

L'hydrolyse du cétal est réalisé par dissolution dans 600 ml d'une solution d'acide chlorhydrique normale et repos de 18 heures à la température ambiante.

On rend ensuite le milieu basique par addition de soude à pH = 10, filtre, lave à l'eau et sèche le précipité.

On obtient ainsi 25 g de cristaux blancs qui sont recristallisés dans l'acétonitrile. Fi = 165°, solidification et fusion à 185° (double PF).

**Exemple 15**

1-[4-p. fluorophényl 4-hydroxy)-1 butyl] 4-(2-amino S-triazol-5 yl aminométhyl) pipéridine

A une solution de 3,9 g de 1-[(4-p. flurophényl 4-oxo)-1 butyl] 4[(2-amino S-triazol-5 yl) aminométhyl] pipéridine, dans 100 ml de méthanol refroidi à 10°, on ajoute en agitant 1 g de borohydrure de sodium par petites portions.

Après 1h 30 d'agitation à la température ambiante, il y a apparition de cristaux qui sont filtrés, lavés à l'eau et à l'éthanol.

Après recristallisation dans l'éthanol, on obtient 2,1 g du produit cherché sous forme de cristaux blancs de PFi = 210°.

**Exemple 16**

1-(4-p. fluorophényl 4-oxo)-1 butyl 4-(2-amino 1,3,5-oxadiazol-4 yl aminométhyl) pipéridine

On porte 17 heures à l'ébullition un mélange de 1-[(4-p. fluoraphényl 4,4-éthylènedioxy)-1 butyl] 4-(N-cyano S-méthylisothiouréîdométhyl) pipéridine, 3,6 g de chlorhydrate d'hydroxylamine, 4,2 g de bicarbonate de sodium et de 100 ml d'éthanol.

Après filtration, on concentre à sec le cétal formé qui se présente sous forme d'une huile vitreuse qui est mise en solution dans 200 ml d'acide chlorhydrique normal, afin d'hydrolyser la fonction cétal.

Après 20 heures à la température ambiante, cette solution est rendue basique par la soude et extraite au chloroforme.

L'huile obtenue après évaporation du solvant est purifiée par passage sur une colonne de silice H. Merck en

utilisant le melange chloroforme 9-isopropylamine 1 comme éluant.

On obtient ainsi 2,2 g d'huile qui cristallise par traitement à l'acétonitrile.

Le produit cherché est obtenu sous forme de cristaux blancs de PFi = 134°.

**Exemple 17**

1-[4-(p. fluorophényl 4,4-éthylènedioxy)-1 butyl] 4-(2-amino 3-méthyl S-triazol-5 yl aminométhyl) pipéridine et 1-[(4-p. fluorophényl 4,4-éthylènedioxy)-1 butyl]-4-(2-amino 4-méthyl S-triazol-5 yl aminométhyl) pipéridine

On porte 18 heures à l'ébullition un mélange de 40 g de 1-[(4-p. fluorophényl 4,4-éthylènedioxy)-1 butyl] 4-(N-cyano S-méthylisothiouréîdométhyl) pipéridine et de 100 ml de méthyl-hydrazine dans 300 ml d'éthanol.

Après évaporation à sec, on obtient 47 g d'une huile qui est redissoute à chaud dans 250 ml d'autonitrile. Cette solution cristallise après une nuit de repos. Les cristaux sont séparés, filtrés, lavés à l'acétonitrile et séchés.

On obtient 14 g d'un produit incolore fondant à 100° et homogène en chromatographie couche mince (Rf = 0,25). Les liqueurs mères sont concentrées à sec. Le résidu sec huileux montre deux taches en chromatographie couche mince l'une de RF 0,25 et l'autre de Rf 0,1.

Les deux composés sont séparés par chromatographie sur colonne de silice H. Merck 1,600 kg en employant le solvant chloroforme - isopropylamine comme éluant.

**Exemple 18**

1-[(4-p. fluorophényl 4-oxo)-1 butyl] 4-(2-amino 4-méthyl S-triazol-5 yl aminométhyl) pipéridine

12,5 g de 1-[(4-p. fluorophényl-4,4 éthylènedioxy) 1-butyl] 4-[(2-amino 4-méthyl S-triazol-5 yl) aminométhyl] pipéridine sont mis en solution dans 500 ml d'acide chlorhydrique N et laissés 17 heures à la température ambiante.

On rend alcalin le milieu, ensuite, par la soude et on épuise à l'acétate d'éthyle.

Après lavage à l'eau de la phase organique, séchage et concentration, on obtient une huile qui traitées à l'acétonitrile fournit 3,9 g de cristaux blancs.

La 1-[(4-p. fluorophényl 4-oxo)-1 butyl] 4-[(2-amino 4-méthyl S-triazol-5 yl) aminométhyl] pipéridine fond à 114-115°.

**Exemple 19**

1-[(4-p. fluorophényl 4-oxo)-1 butyl] 4-[2-amino 3-méthyl S-triazol-5 yl) aminométhyl] pipéridine

On prépare une solution de 3,5 g de 1-(4-p. fluorophényl 4,4-éthylènedioxy)-1 butyl 4-[(2-amino 3-méthyl S-triazol-5 yl) aminométhyl] pipéridine dans 100 ml d'acide chlorhydrique normal.

Après 18 heures à la température ambiante, on alcalinise par la soude et extrait à l'acétate d'éthyle la solution aqueuse. Après concentration du solvant on obtient un solide qui est recristallisé dans l'acétonitrile.

On obtient ainsi 1,3 g du produit cherché sous forme de cristaux de Fi = 162°.

**Exemple 20**

1-(4-p. fluorophényl 4-oxobutyl-1) 4[(2-amino 4-méthyl 1,3,4-triazolyl-5 amino)méthyl] pipéridine

**Stade A**

On chauffe à reflux pendant 30 mn 10g65 de benzaldéhyde et 4g6 de méthylhydrazine dans 100 ml d'éthanol. Après concentration à sec on obtient la N-méthylhydrazone du benzaldiohyde sous forme d'une huile claire. On la reprend par 100 ml d'éthanol et on y ajoute 14g6 de N-cyeno iminodithiocarbonate de méthyle. On porte deux heures au reflux puis on distille le solvant à sec. On recueille un résidu huileux qui cristallise par trituration avec l'éther isopropylique.

Après filtration, les cristaux sont lavés puis séchés, on obtient ainsi la (cyanoimino) (méthylthio) méthyl N-méthyl N'- benzylidényl hydrazine sous forme de cristaux incolores fondant à 114°.

**Stade B**

On porte 2 heures à l'ébullition un mélange de 3g25 de l'hydrazine du stade A, 4g5 de 1-(p. fluorophényl 4,4-éthylènedioxy butyl-1) 4-(aminométhyl) pipéridine dans 50 ml d'éthanol. On évapore ensuite le solvant à sec. L'huile orangée obtenue est redissoute dans 100 ml d'acide chlorhydrique 2N.

La solution acide est portée à l'ébullition pendant 15 mn puis laissée reposer pendant 18 heures à température ambiante. La solution est ensuite épuisée à l'éther pour extraire le benzaldéhyde. La phase aqueuse est séparée puis rendue alcaline par addition de lessive de soude.

On épuise alors à l'acétate d'éthyle. La phase organique est lavée à l'eau, séchée et évaporée à sec. Le résidu huileux pesant 4g75 est trituré avec un peu d'éther d'où il cristallise.

Le dérivé triazolique est ensuite recristallisé de l'acétonitrile en quantité minimale. On obtient ainsi 1g4 de produit sous forme de cristaux F = 96°.

Le produit montre un spectre IR identique à celui de l'isomère F = 114-115° obtenu par réaction directe avec la méthylhydrazine. De même en chromatographie en couche mince les Rf dans le système chloroforme-isopropylamine 9 : 1 sont identiques.

Les différences entre les points de fusion trouvent leur explication dans le fait que la micro-analyse montre pour le produit F 96° la cristallisation avec une molécule d'eau.

Le composé fondant à 162° est de ce fait indubitablement le dérivé (2-amino 3-méthyl S-triazolyl-5) aminé.

**Exemple 21**

1-(indol-3 yl éthyl) 4-(2-amino S-triazolyl-5 amino) pipéridine

**Stade A**

1-(indol-3 yl éthyl) 4-acétylaminopipéridine

On maintient sous agitation à température ambiante pendant 20 heures un mélange de 18g7 de 4-acétylaminopipéridine, de 29g5 de 3-(2-bromoéthyl) indole et de 13g3 de triéthylamine préalablement dissoute dans 70 ml de diméthylformamide. On concentre ensuite à sec et le résidu est repris par le minimum d'eau contenant quelques ml d'ammoniaque puis on épuise la phase aqueuse au chloroforme.

La solution chloroformique est lavée à l'eau, séchée puis concentrée. On obtient ainsi 30 g du produit cherché sous forme de cristaux incolores fondant à 160°.

Après purification par recristallisation de l'acétonitrile, on obtient un produit spécimen fondant à 165°.

**Stade B**

1-(indol-3 yl éthyl) 4-aminopipéridine.

On dissout 26 g de 1-(indol-3 yl éthyl) 4-acétyl aminopipéridine obtenue au stade A, dans 260 ml d'acide chlorhydrique 2N puis on amène progressivement à l'ébullition que l'on maintient pendant 2 heures trente. Après refroidissement on filtre le milieu et alcalinise le filtrat par addition de soude concentrée. Il apparait un précipité huileux qui se concrétise rapidement en cristaux.

On sépare les cristaux qu'on essore, rince à l'eau et sèche. On obtient ainsi 23 g de l'aminopipéridine sous forme de cristaux crème F = 105-110°

En chromatographie en couche mince le produit est homogène (une seule trace de Rf 0,45) en utilisant le système méthanol-isopropylaminé 9 : 1 comme solvant d'élution. Le composé est assez pur pour pouvoir être utilisé pour le stade suivant de la synthèse.

**Stade C**

1-(indol-3 éthyl) 4-[(N-cyano S-méthyl isothiouréîdo)] pipéridine

On ajoute 24 g de 1-(indol-3 yl éthyl) 4-amino-pipéridine à un mélange de 13g25 de N-cyanoimino isothiocarbonate de diméthyle et de 300 ml d'éthanol.

On porte 4 heures à l'ébullition puis on sépare l'insoluble que l'on triture avec de l'éther. Les cristaux ainsi formés sont filtrés, essorés puis lavés et séchés. On obtient ainsi 27 g d'isothiourée (F = 44°).

**Stade D**

1[(indol-3 yl éthyl) 4-(2-amino S-triazolyl-5 amino) pipéridine

On dissout dans 100 ml d'éthanol 6g8 de 1-(indol-3 yl éthyl) 4-(N- cyano S-méthyl isothiouréîdo) pipéridine et 4m15 d'hydrate d'hydrazine. On porte 20 heures le mélange au reflux puis on évapore à sec le solvant. On obtient une huile orangée, qui remise en solution dans l'acétonitrile cristallise après quelques heures.

On sépare les cristaux qu'on rince, lave à l'éther et sèche. On obtient ainsi 5g6 de triazole désiré. On purifie celui-ci par recristallisation de l'éthanol. PF = 200°. Le produit est cristallisé avec 1/2 molécule d'eau.

**Exemple 22**

1-(indol-3 yl éthyl) 4-[(2-amino S-triazolyl-5 amino) méthyl] pipéridine

**Stade A**

1-(indol-3 yl éthyl) 4-pipéridino carboxamide

On met en suspension dans 200 ml de diméthyl formamide à température ordinaire 22g4 de 3-(bromo-2 éthyl) indole, 12g8 de pipéridine-4 carboxamide et 10g1 de triéthylamine.

Après 18 heures d'agitation, le mélange est concentré à sec et le résidu huileux est triturée avec de l'eau ammoniacale jusqu'à début de cristallisation. Après une nuit de repos en glacière, les cristaux sont séparés par filtration, lavés à l'eau puis à l'hexane et séchés. Après recristallisation de l'éthanol, on obtient 19 g d'indolyléthyl pipéridino carboxamide sous forme de cristaux incolores fondant à 184°.

En chromatographie en couche mince le produit est homogène (une tache de Rf 0,70 avec le système éluant méthanol-isopropylamine 9 : 1).

**Stade B**

1-(indol-3 yl éthyl) 4-aminométhyl pipéridine

On prépare une suspension de 10 g d'aluminohydrure de lithium et d'aluminium dans 150 ml de tétrahydrofuran sous agitation et on y ajoute, tout en maintenant l'agitation et en évitant toute montée en température, 20 g de 1-(indol-3 yl éthyl) pipéridino-4 carboxamide. Une fois l'addition terminée on chauffe le mélange pendant 4 heures à ébullition du solvant.

L'excès de réactif est détruit par addition lente d'eau en maintenant un refroidissement énergique pour éviter que la température du milieu ne monte au dessus de 0°.

Après filtration sur une argile vendue sous la dénomination CELITE, la solution est évaporée à sec. On recueille un résidu huileux que l'on reprend par le minimum d'éthanol, filtre et concentre à nouveau à sec.

On obtient ainsi 19 g de l'aminométhylpipéridine sous forme d'un produit liquide qui cristallise progressivement.

Le produit présente un double point de fusion : 80° puis après résolidification, fusion à 130-132°.

**Stade C**

1-(indol-3 yl éthyl) 4-(N-cyano S-méthyl isothiouréîdométhyl) pipéridine.

On chauffe pendant 4 heures à l'ébullition du solvant un mélange de 18g8 de 1-(indol-3 yl éthyl) 4-aminométhyl pipéridine et de 10g7 de N-cyano imino dithiocarbonate de méthyle dans 300 ml d'éthanol.

On laisse refroidir puis on concentre à sec. Le produit huileux isolé est repris par un peu d'éther jusqu'à

début de cristallisation. On laisse reposer les cristaux qu'on sépare par filtration, essore, rince à l'éther et sèche. On recueille ainsi 18 g de 1-(indol-3 yl éthyl) 4-(N-cyano S-méthyl isothiouréîdométhyl) pipéridine sous forme de cristaux incolores fondant à 150°.

**Stade D**

1-(indol-3 yl éthyl) 4-[(2-amino S-triazolyl S-amino) méthyl] pipéridine

On porte 18 heures à l'ébullition du solvant une solution de 7g1 de 1-(indol-3 yl éthyl) 4-(N-cyano S-méthyl isothiouréîdométhyl) pipéridine et de 4 ml d'hydrate d'hydrazine dans 100 ml d'éthanol.

Après concentration à sec, le résidu est repris par l'acétonitrile d'où le produit cristallise. On laisse la suspension reposer quelques heures puis on filtre, lave les cristaux à l'acétonitrile et les sèche sous vide. On obtient ainsi 6g3 du triazole cherché. Il se présente sous forme de cristaux incolores fondant à 172°.

**Exemple 23**

En opérant selon le mode opératoire de l'exemple 1 ou de l'exemple 14 on a préparé les composés suivants:
1-[4-(p. fluorophényl) 4-oxobutyl-1] 4-(2-amino 1,3,4-triazolyl-5) aminopipéridine F = 216°
1-[4-(p. fluorophényl) 4-oxobutyl-1] 4-(2-amino 1,3,5-oxadiazolyl-4) aminopipéridine F = 186°
1-[4-(p. fluorophényl) 4-oxobutyl-1[ 4-(2-amino 4-méthyl 1,3,4-triazolyl-5) aminopipéridine F = 152°
1-[4-(p. fluorophényl) 4-oxobutyl-1] 4-(2-amino 3-méthyl 1,3,4-triazolyl-5) aminopipéridine F = 162°
1-[2-(indolyl-3) éthyl] 4-[(2-amino 1,3,4-oxadiazolyl-5) aminométhyl] pipéridine

**Exemple 24**

Comprimés à 2mg5 de principe actif
1-[4(p. fluorophényl) 4-oxobutyl)-1] 4-(2-amino 1,3,4-oxadiozolyl-5 méthylamino) pipéridine 25 g

| | |
|---|---|
| Amidon de blé | 650 g |
| Lactose | 175 g |
| Cellulose microcristalline | 45 g |
| Phosphate dicalcique | 900 g |
| Stéarote de magnésium | 25 g |
| Carbocyméthylamidon | 17 g 5 |

pour 10.000 comprimés terminés au poids moyen de 0g185.

**Exemple 25**

Comprimés à 5 mg de principe actif
1-(benzodioxanne-2 yl méthyl) 4-(2-amino 1,3,4-triozolyl-5 méthylamino) pipéridine 50 g

| | |
|---|---|
| Amidon de blé | 400 g |
| Amidon de maîs | 220 g |
| Cellulose microcristalline | 375 g |
| Sulfate de calcium | 510 g |
| Carboxyméthyl cellulose | 20 g |
| Ethylcellulose | 15 g |

pour 10.000 comprimés finis au poids moyen de 0g150.

**Exemple 26**

Etude pharmacologique des composés selon invention

a) Détermination de la toxicité aiguë

Une dose léthale moyenne ($DL_{50}$) approchée a été déterminée après administration par voie orale des composés, selon l'invention, à doses croissantes à des lots de 10 Souris femelles EOPS d'élevage CESAL par la méthode de D.E.J. Campbell et W. Richter (Acta Pharm. and Toxicol. 25 (1967) 345). Les animaux sont gardés en observation pendant 5 jours. Les morts, quand il y en a, sont dénombrés. Les résultats montrent que les doses léthales moyennes s'échelonnent de 360 mg/kg à 1200 mg/kg selon les composés.

b) Détermination de l'effet anti-hypertensif

L'essai est réalisé sur des lots de Rats mâles vigiles rendus hypertendus par ligature de l'aorte abdominale.

Les produits, selon l'invention, sont administrés par voie orale aux doses de 1,2 et 5 mg/kg. Ils entraînent une baisse de pression nette et prolongée.

Par ailleurs, les composés, selon l'invention, administrés par voie intraveineuse aux Rots normo-tendus ou aux chions normotendus sous anesthésie à des doses variant de 2 à 10 µg/kg pour les composés les plus actifs et de 20 à 50 µg/kg pour les autres composés, entraînent une hypotension très marquée.

**Recherche d'un effet vasodilatateur**

Les composés, selon l'invention, ne provoquent pas d'effet vasodilatateur périphérique, On ne constate aux doses administrées aucune vaso-dilatation nin aucune augmentation de la température cutanée des pattes postérieures du Rat.

**Revendications**

1. Les 2-amino 5-[(aralcoyl pipéridino alcoyl) amino 1,4-diazoles] de formule générale I

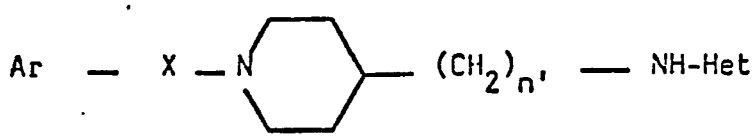 (I)

dans laquelle $A_R$ est un radical aryle mono-cyclique choisi dans le groupe constitué par la pyridine, l'oxazine, la pyrazine et un radical phényle de formule générale

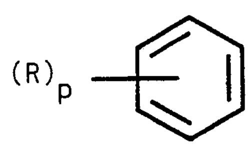

dans laquelle R est de l'hydrogène, un radical alcoyle en $C_1$-$C_6$, un radical alcoxy en $C_1$-$C_6$, un halogène, un trifluorométhyle, un radical sulfonamido, un radical trifluorométhoxy, un radical cyano, un radical carboxy, un radical carbalcoxy, un radical carboxamido, un groupe nitro ou un groupe alcoylène-dioxy en $C_1$-$C_6$ et p est un nombre entier variant de 1 à 3;

ou un radical aromatique bicyclique non saturé ou partiellement saturé choisi dans le groupe constitué par le tétrahydronaphtyle, le naphtyle-1, le naphtyle-2, le benzodioxanyle, le benzodioxényle, le quinoléinyle, le thiachromanyle, l'indolyle-2 et l'indolyle-3.

X est une chaîne alcoylène, éventuellement substituée, choisie dans le groupe constitué par un radical $(CH_2)_n$; un radical $CHOH-(CH_2)_{n_1}$; un radical $CO-(CH_2)_{n_1}$, et un radical $-\underset{\underset{K}{\|}}{C}-(CH_2)_{n_1}$,

pour lesquels n est égal à 1, 2, 3 ou 4
$n_1$ est égal à 1, 2 ou 3
n' est égal à 0, 1 ou 2

K est un groupe 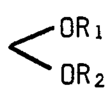

dans lequel

$R_1$ et $R_2$ sont des radicaux alcoyle inférieur ayant de 1 à 6 atomes de carbone identiques ou différents - ou une chaîne alcoylène inférieur ayant de 1 à 4 atomes de carbone et Het est un radical hétérocyclique choisi

dans le groupe constitué par
- les aminotriazoles de formule A

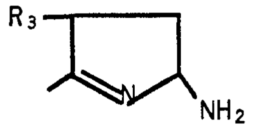

(A)

dans laquelle $R_3$ est de l'hydrogène, un radical alcoyle en $C_1$-$C_6$, un radical aryle ou un radical aralcoyle inférieur dans lequel la chaîne alcoyle a de 1 à 6 atomes de carbone;
- et les 1,4-diazoles de formule B

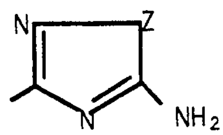

(B)

dans laquelle Z est un radical -NH-, $NR_3$ ou de l'oxygène ($R_3$ ayant les mêmes significations que précédemment), le terme aryle désignant un radical aromatique monocyclique, homo ou hétérocyclique, éventuellement substitue par 1, 2 ou 3 substituants choisis dans le groupe constitué par un alcoyle inférieur, un halogène, un alcoxy inférieur, un alcoylène-dioxy et trifluorométhyle.

2. Les sels d'addition avec un acide minéral ou organique des composés selon la revendication 1.

3. Les formes optiquement-actives des composés selon la revendication 1 ou la revendication 2.

4. Les composés selon l'une des revendications précédentes répondant à la formule générale $I_A$

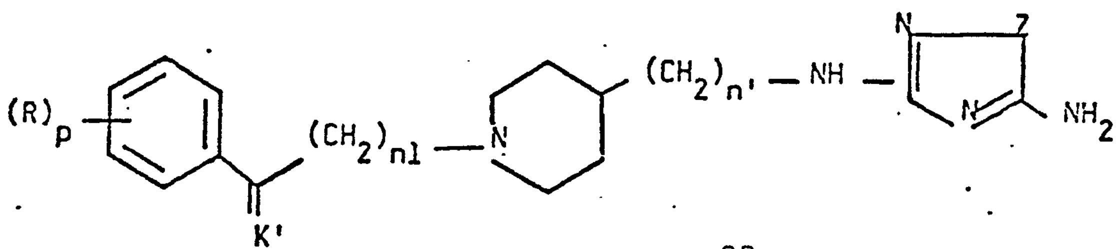

$(I_A)$

dans laquelle K' représente deux hydrogènes, un groupe 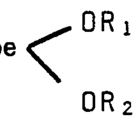

dans lequel $R_1$ et $R_2$ sont définis comme précédemment, un groupe 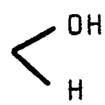

ou l'oxygène d'une fonction cétone R, p, $n_1$ et n' sont définis comme précédemment et Z représente un groupe NH, $NR_3$ ou de l'oxygène ($R_3$ étant défini comme précédemment).

5. Les composés selon l'une des revendications 1 à 4 répondant à la formule générale $I_B$

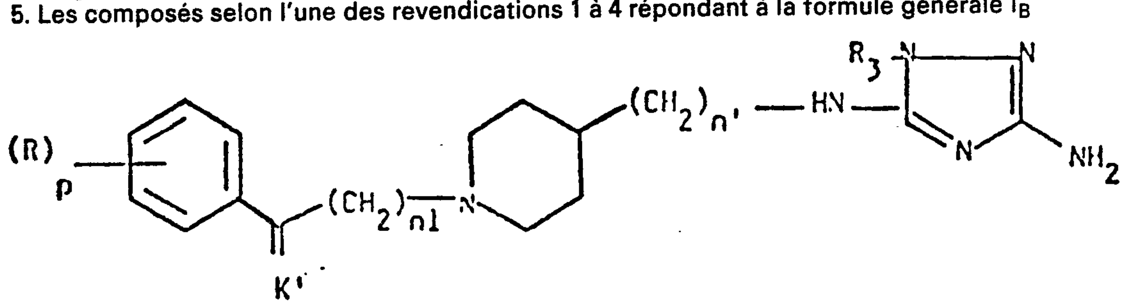

$(I_B)$

dans laquelle les substituants R, $R_3$, K', p, $n_1$ et n' ont les significations fournies précédemment.

6. Les composés selon l'une des revendications 1 à 4 répondant à la formule générale $I_C$

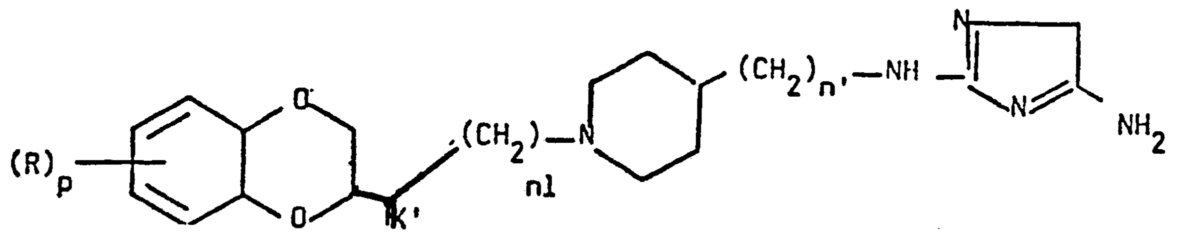

$(I_C)$

dans laquelle les substituants R, K', Z, p, n', $n_1$ ont les significations fournies antérieurement.

7. Les composés selon l'une des revendications 1 à 4 répondant à la formule générale $I_D$

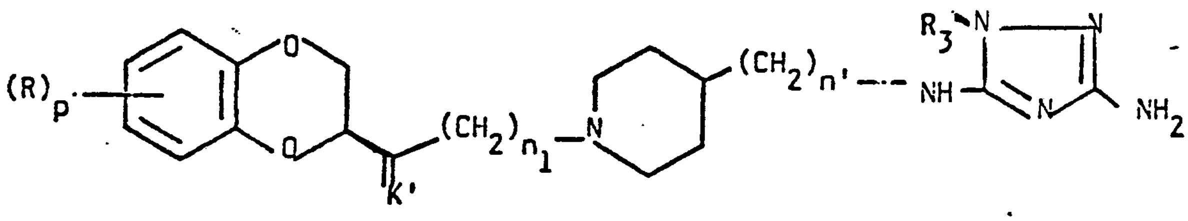

$(I_D)$

dans laquelle les substituants R, K', p, $n_1$ et $R_3$ ont les mêmes significations que précédemment.

8. Les composés selon l'une des revendications 1 à 4 répondant à la formule générale $I_E$

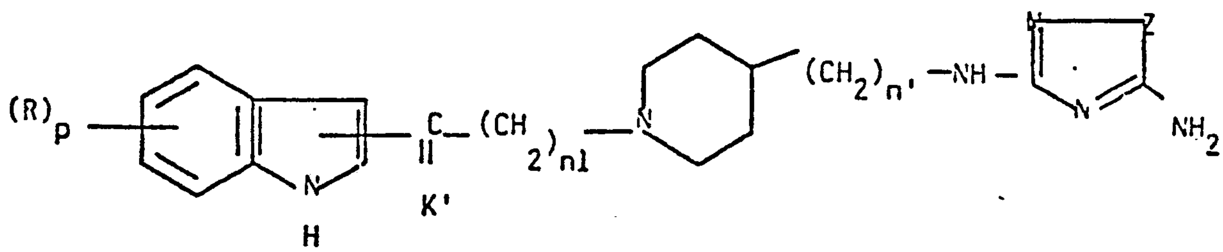

$(I_E)$

dans laquelle les substituants R, K', p, Z, $n_1$ et n, sont définis comme précédemment.

9. Un composé selon la revendication 1 ou la revendication 2 à savoir la 1-[(4-p. fluorophényl)4-oxobutyl-1] 4-(2-amino 1,3,4-triazolyl-5) aminométhyl pipéridine et son chlorhydrate.

10. Les compositions pharmaceutiques renfermant à titre de principe actif au moins un composé selon l'une des revendications 1 à 9 en association ou en mélange avec un excipient ou un véhicule inerte non-toxique, pharmaceutiquement acceptable.

11. Les compositions selon la revendication 10 présentées sous l'une des formes convenant pour l'administration par voie parentérale, buccale, rectale, sublinguale ou percutanée.

12. Une composition pharmaceutique selon l'une des revendications 10 ou 11 dans laquelle la quantité de principe actif s'échelonne de 0,1 à 50 mg par prise.

13. Un procédé d'obtention des composés selon l'une des revendications 1 à 9 caractérisé en ce que l'on soumet une N-cyano isothio- ou isourée de formule générale II

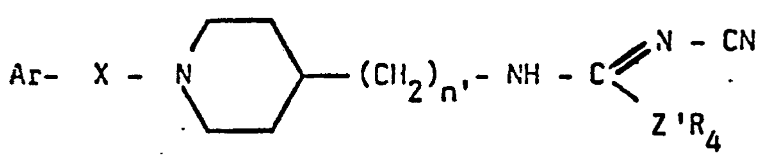

(II)

dans laquelle $A_R$, X et n' ont les définitions fournies antérieurement

Z' est de l'oxygène ou du soufre

et $R_4$ est un radical alcoyle en $C_1$-$C_6$

à l'action d'un dérivé aminé de formule générale III

$HZ - NH_2$ (III)

dans laquelle Z est défini comme précédemment

pour former, lorsque Z est de l'oxygène, un 2-amino 3-oxa 1,4- diazole de formule générale I'

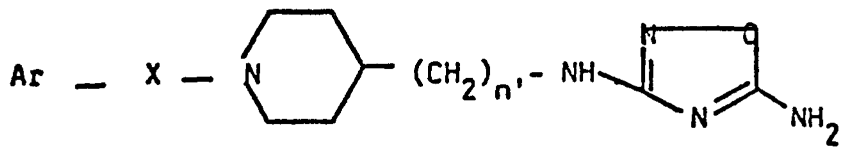

(I')

ou - lorsque Z est un radical imino - un 2-amino 1, 3, 4-triazole de formule I''

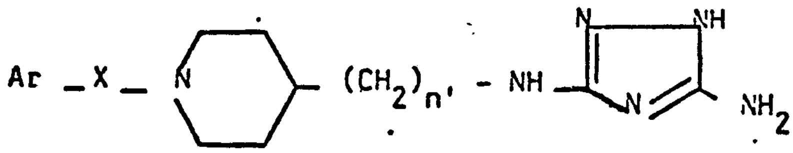

(I'')

ou - lorsque Z est un radical $NR_3$ - un mélange de 2- amino 1, 3, 4-triazoles substitués de formule

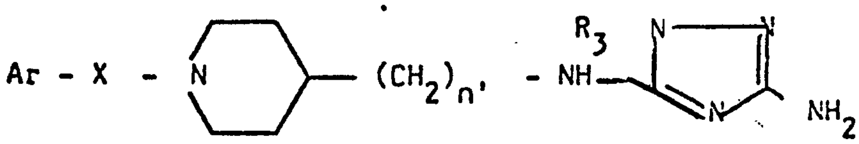

et

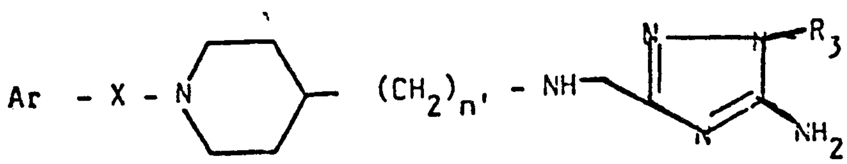

que l'on sépare par des moyens physiques.

14. Un procédé selon la revendication 13 qui comporte l'étape supplémentaire de salification par addition d'un acide minéral ou organique.

15. Un procédé selon la revendication 13 ou la revendication 14 qui comporte l'étape supplémentaire de dédoublement par salification à l'aide d'un acide optiquement-actif.

**Patentansprüche**

1. Die 2-Amino 4-[aralkyl piperidino alkyl) amino 1,4-diazolen] der allgemeinen Formeln I

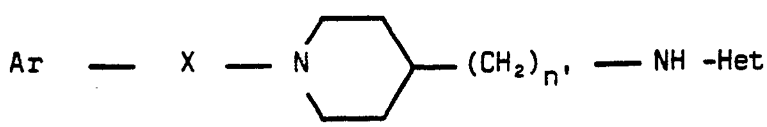

(I)

worin $A_R$ ein mono-cyclisch aryl Radikal aus der Gruppe gewählt ist, die Pyridin, Oxazin, Pyrazin und Phenyl der allgemeinen Formeln

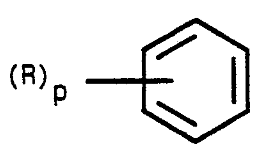

worin R Wasserstoff, ein niedrig Alkyl Radikal, ein niedrig Alkoxy Radikal, ein Halogen, ein Trifluoromethyl, ein Sulfonylamino, ein Trifluoromethoxy, ein Cyan, ein Carboxy Radikal, ein Carboxamido Radikal, eine Nitro Gruppe, oder eine Alkylene dioxy Gruppe,

und p ein ganze Zahl von 1 bis 3 ist,

enthält

oder ein bicyclisch aromatische ungesätigte oder partiell ungesättigte Radikal aus die Gruppe gewählt ist, die Tetrahydronaphtyl, Naphtyl-1, Naphtyl-2, Benzodioxanyl, Benzodioxenyl, Chinoleinyl, Thiachromanyl,

Indolyl-2 und Indolyl-3 enthält.

X eine gegebenenfalls substituierte Alkylene-Kette aus die Gruppe gewählt ist, die ein Radikal $(CH_2)_{n'}$, ein Radikal $(CHOH)-(CH_2)_{n_1}$, ein Radikal $CO-(CH_2)_{n_1}$ und ein Radikal - $C-(CH_2)_{n_1}$ - enthält
||
K

wofür n gleich als 1, 2, 3 oder 4 ist
$n_1$ gleich als 1, 2, oder 3 ist
n' gleich als 0, 1 oder 2 ist
K eine gruppe 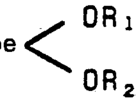

ist,

worin $R_1$ und $R_2$ $C_1$-$C_6$ niedrig Alkyl Radikale, die identische or verschiedene sind, oder eine niedrig Alkylene Kette mit 1 bis 4 Kohlenstoffatomen ist

und Het ein heterocyclisch Radikal ist, der aus der Gruppe gewählt ist, die

- die Aminotriazole der Formel A

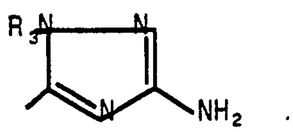

(A)

worin $R_3$ ein Wasserstoff, ein niedrig-Alkyl Radikal, ein Aryl Radikal oder ein Aryl-(niedrig Alkyl) Radikal ist, in welchem die Alkyl Kette von 1 bis 6 Kohlenstoffatomen enthält

- und die 1,4-Diazole der Formel B

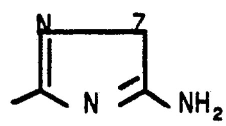

(B)

worin Z ein -NH- Radikal, ain $N-R_3$ Radikal, oder ein Sauerstoff bedeutet ($R_3$ hat dieselbe Bedeutungen als vorher) enthält.

2. Die mit einer inorganischen oder organischen Säure Additions Salze der Verbindungen nach Anspruch 1.

3. Die optisch - aktive Isomeren der Verbindungen nach Anspruch 1 oder Anspruch 2.

4. Die Verbindungen nach einer dar vorstehanden Ansprüche die die Formel $I_A$ haben

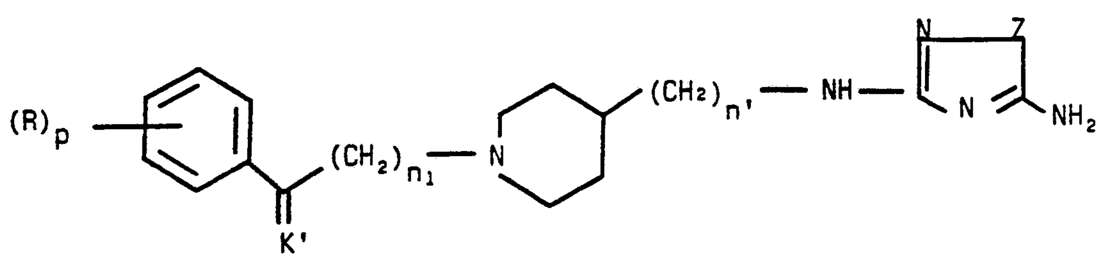

$(I_A)$

worin K zwei Wassarstoffatomen, die Gruppe 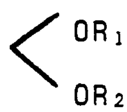

in welchen $R_1$ und $R_2$ wie vorher definiert sind, eine Gruppe 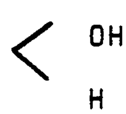

oder ein Sauerstoffatom einer Keton
Funktion bedeutet

R, p, $n_1$ und n' wie vorher definiert sind und Z eine Gruppe NH odar $NR_3$ oder ein Sauerstoffatom darstellt ($R_3$ ist wie vorher definiert).

5. Die Verbindungen nach einer der Ansprüche 1 bis 4 der allgemeinen Formeln $I_B$

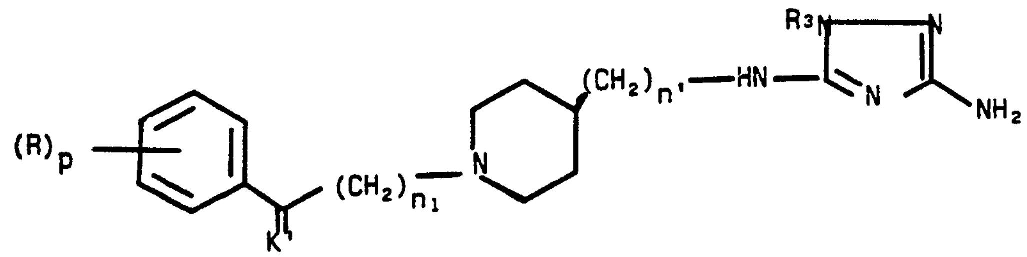

$(I_B)$

in der die Substituenten R, $R_3$, K', p, $n_1$ und n' die vorherigen Bedeutungen besitzen.

6. Die Verbindungen nach einer der Ansprüche 1 bis 4 der allgemeinen Formeln $I_C$

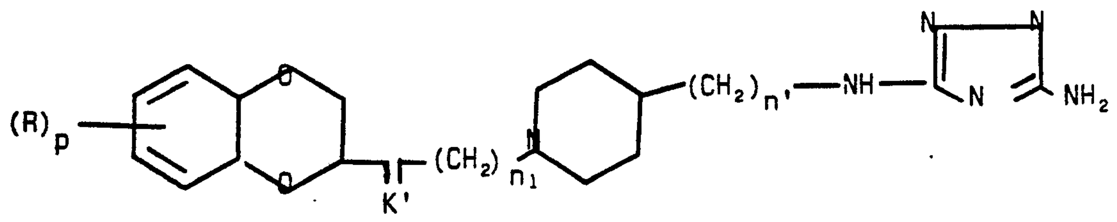

$(I_C)$

in der die Substituenten R, K', Z, p, n' und $n_1$ die vorherigen Bedeutungen besitzen.

7. Die Verbindungen nach einer der Ansprüche 1 bis 4 der allgemeinen Formeln $I_D$

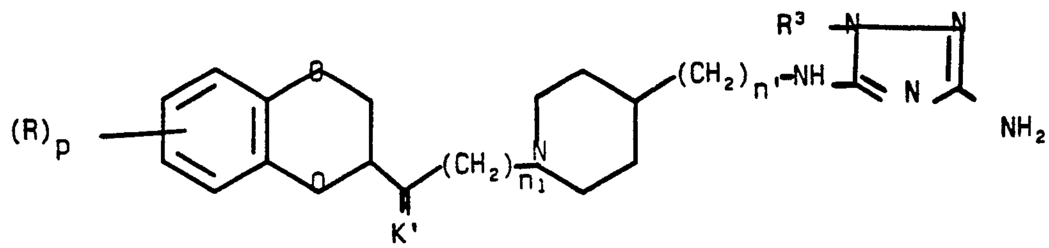

$(I_D)$

in der die Substituenten R, K', p, n' $n_1$ und die vorherigen Bedeuttungen besitzen.

8. Die Verbindungen nach einer der Ansprüche 1 bis 4 der allgemeinen Formeln $I_E$

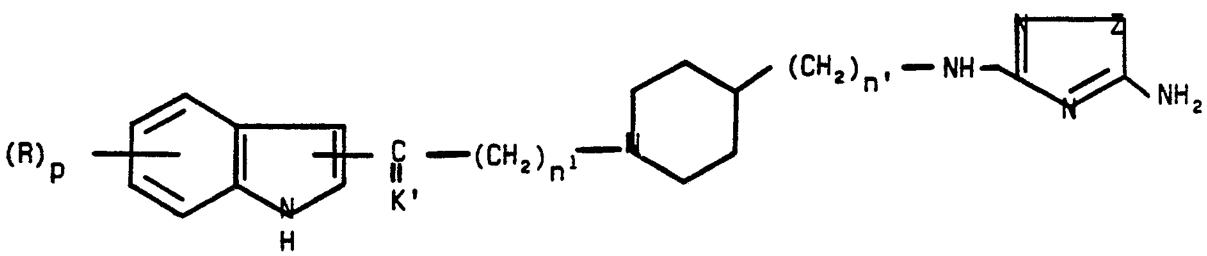

$(I_E)$

worin die Substuenten R, K', p, Z, $n_1$ und n' wie vorher definiert sind.

9. Eine Verbindung nach Ansprüch 1 oder Ansprüch 2 nämlich 1-[(4-p. fluorophenyl) 4-oxobutyl-1] 4-(2-amino 1,3,4-triazolyl-5) aminomethyl Piperidine und deren Chlorhydrats.

10. Die Pharmazeutische Zusammensetzungen enthaltende als Wirkstoffe, mindenstens eine Verbindung nach einer der Ansprüche 1 bis 9 in Verbindung oder Gemisch mit einem inerten, non-toxischen pharmazeutisch verträglichen Bindemittel oder Vehikeln.

11. Die pharmazeutische Zusammansetzungen nach Ansprüch 10 in einer Form dargestellt die für parenteral, oral, rektal, sublingual oder percutan Verabreichung geeignet ist.

12. Eine pharmazeutische Zusammansetzung nach einer der Ansprüche 10 oder 11 in der, die Menge Wirkstoff zwischen 0.1 und 50 mg per Einzelheit erstreckt.

13. Verfahren zur Herstellung der Verbindungen nach einer der Ansprüche 1 bis 8 dadurch gekennzeichnet das ein N-Cyan isothio- oder isoharnstoff der allgemeinen Formeln II

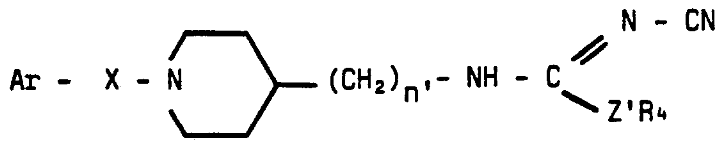

(II)

worin $A_R$, X und n' die vorherigen angegebene Bedeutungen besitzen

Z' Sauerstoff oder Schwefel ist und $R_4$ eine niedrig Alkyl Radikal ist mit einem Aminoderivate der allgemeinen Formeln III

$HZ - NH_2$ (III)

worin Z wie vorher definiert ist umsetzt, um wenn Z eine Sauerstoffatom ist, ein 2-amino 3-oxa 1,4-diazol der allgemeinen Formeln I' zu erzeugen

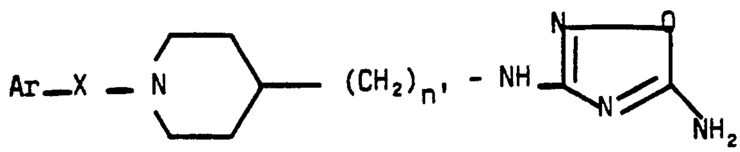

(I')

oder wenn Z eines Iminoradikal ist, ein 2-amino 1,3,4-Triazol der allgemeinen Formeln I''

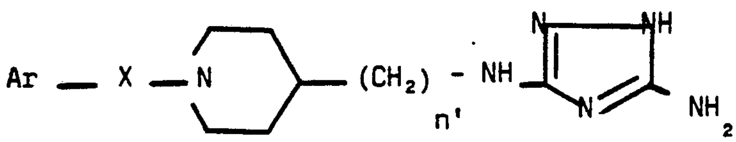

(I")

zu erzeugen

oder wenn Z eines Radikal $R^3$ ist, ein Gemisch von substituerten 2-amino 1,3,4-Triazole der allgemeinen Formeln

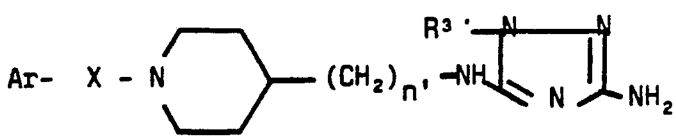

und

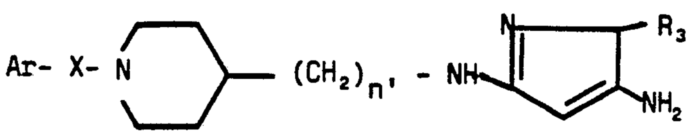

zu erzeugen, die durch physikalische Methoden getrennet werden.

14. Verfahren nach Anspruch 13 das der zusätzliche Stufe von Salzmachung durch Addition einer inorganischen oder organischen Saüre einschliesst.

15. Verfahren nach Anspruch 13 oder Anspruch 14 das der zusätzliche Stufe von optischen Spaltung durch Salzmachung miteiner optisch - aktive Saüre einschliesst.

**Claims**

1. The 2-amino 5-[(aralkylpiperidino alkyl) amino] 1,4 - diazoles of the general formula I

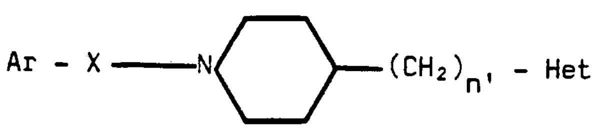

(I)

wherein $A_R$ is a monocyclic aryl radical selected from the group consisting of pyridine, oxazine, pyrazine and phenyl of the general formula

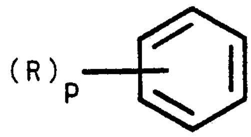

wherein R is hydrogen, a $C_1$-$C_6$ alkyl radical, a $C_1$-$C_6$ alkoxy radical, a halogen, a trifluoromethyl, a sulphonamido, a trifluorométhoxy, a cyano group, a nitrogroup or a $C_1$-$C_6$ alkylenedioxy group

and p is an integer of 1 to 3

or a bicyclic unsaturated or partially saturated aromatic radical selected from the group consisting of tetrahydronaphtyl, naphtyl-1, naphtyl-2, benzodioxanyl, benzodioxenyl, quinoleinyl, thiachromanyl, indolyl-2 and indolyl-3

X is an optionally substituted alkylene chain selected from the group consisting of a $(CH_2)_n$ radical, a CHOH-$(CH_2)_{n_1}$ radical, a CO-$(CH_2)_{n_1}$ radical and a -C -$(CH_2)_{n_1}$ radical.
||
K

in which n is 1, 2, 3 or 4

$n_1$ is 1, 2 or 3

and K is a group 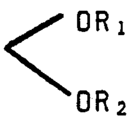

wherein $R_1$ and $R_2$, the same or different, are a $C_1$-$C_6$ lower alkyl radical or $C_1$-$C_4$ lower alkylene chain

and Het is a heterocyclic radical selected from the group consisting of

- the aminotriazoles of formula A

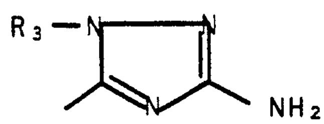 (A)

wherein $R_3$ is hydrogen, a $C_1$-$C_6$ alkyl radical, an aryl radical or an aryl lower alkyl radical in which the alkyl chain has from 1 to 6 carbon atoms and the 1,4-diazoles of formula B

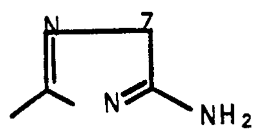 (B)

wherein Z is a -NH-, N-$R_3$ or an oxygen ($R_3$ being defined as previously-given)

and n' is an integer from zero to two.

2. The acid addition salts of the compounds according to claim 1, with a mineral or organic acid.

3. The optically - active isomers of the compounds according to claim 1 or claim 2.

4. The compounds according to any of the preceding claims having the general formula $I_A$

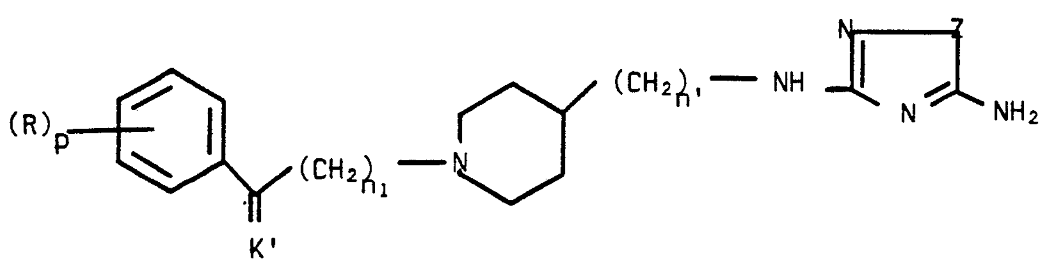 ($I_A$)

wherein K' is two hydrogens, the group 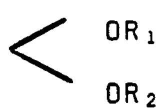

in which $R_1$ and $R_2$ have the previously - given meanings, a group 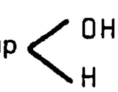

FIG00/10

or the oxygen of ketonic function

R, p, $n_1$, and n' are defined as previously-given and Z is a group -NH-, $NR_3$ or an oxygen ($R_3$ being defined as previously-given).

5. The compounds according to any of the claims 1 to 4 which have the general formula $I_B$

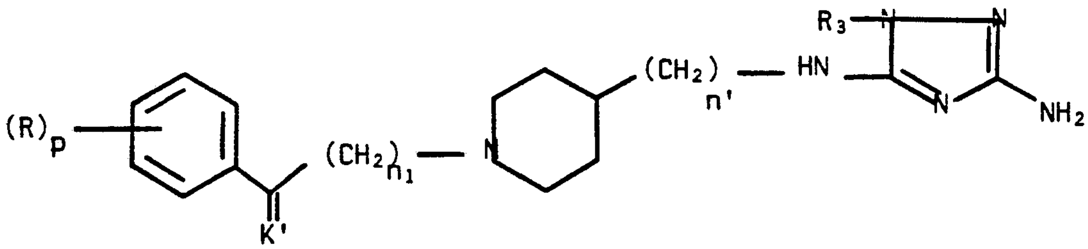

$(I_B)$

wherein the substituents R, $R_3$, K', p, $n_1$ and n' have the above - given definitions.

6. The compounds according to any of claims 1 to 4 which have the general formula $I_C$

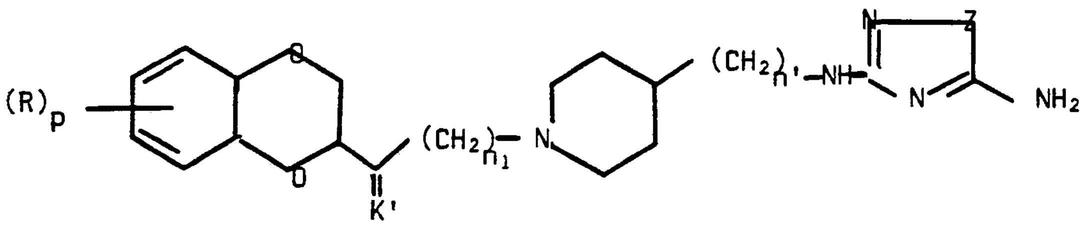

$(I_C)$

wherein the substituents R, K', Z, p, n' and $n_1$ have the above - given definitions.

7. The compounds according to any of the claims 1 to 4 which have the general formula $I_D$

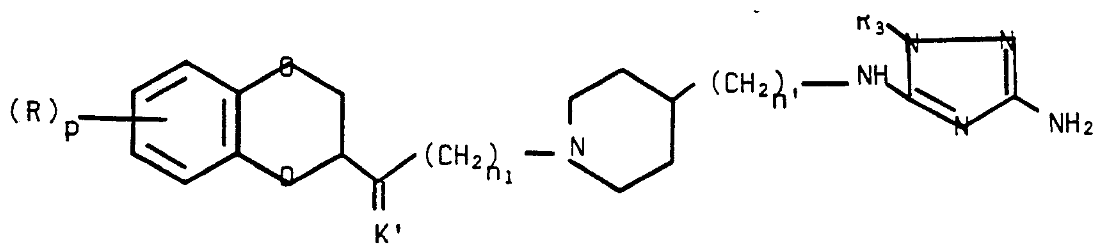

$(I_D)$

wherein the substituents R, K, p, $n_1$ and $R_3$ have the same meanings as previously-given.

8. The compounds according to any of the claims 1 to 4 which have the general formula $I_E$

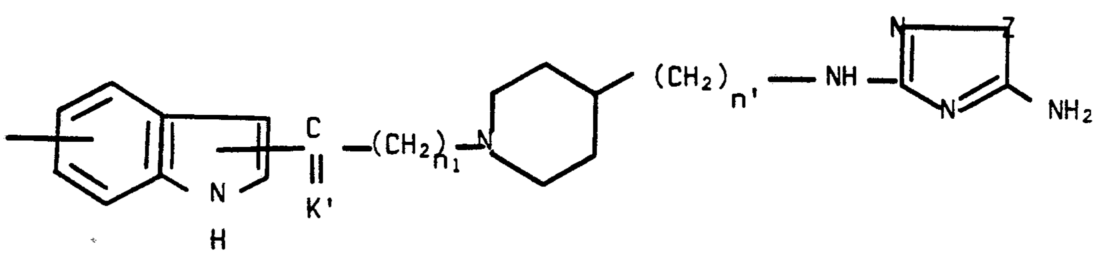

$(I_E)$

wherein the substituents R, K', p, Z, n, and n' have the above - given definitions.

9. A compound according to claim 1 or claim 2 which is 1-[(4-p. fluorophenyl) 4-oxobutyl-1] 4-[(2-amino 2,3,4-triazolyl-5) amino methyl] piperidine and the hydrochloric acid addition salt thereof.

10. The pharmaceutical compositions containing as active ingredient at least one compound according to any one of claims 1 to 9 in admixture or conjunction with an inert non-toxic pharmaceutically - acceptable carrier or vehicle.

11. The pharmaceutical compositions according to claim 10 which are offered in any of the forms suitable for parenteral, oral, rectal, sublingual or percutaneous way of administration.

12. The pharmaceutical compositions according to claim 10 or claim 11 in which the amount of active ingredient ranges from 0.1 to 50 mg per unit dosage.

13. A process for producing the compounds according to any one of the claims 1 to 9 characterized in that a N-cyano isothio- or iso urea of general formula II

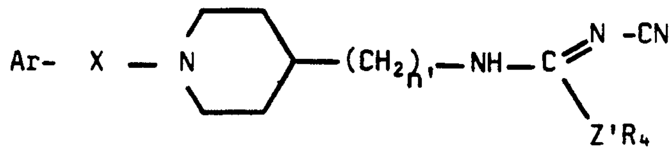

(II)

in which $A_R$, X, Z' is oxygen or sulphur, and n' have the above-given definitions and $R_4$ is a $C_2$-$C_6$ lower alkyl radical is submitted to the action of an aminated derivative of general formula III

$HZ - NH_2$ (III)

wherein Z has the above - given definitions
to produce
when Z is an oxygen, a 2-amino 3-oxa 1,4 diazole of general formula I'

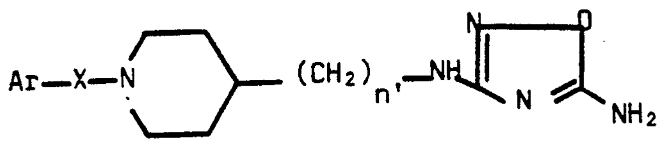

(I')

or when Z is an imino radical, a 2-amino 1,3,4-triazole of formula I''

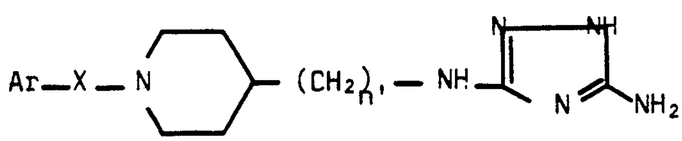

(I'')

or when Z is a N-$R_3$ radical, a mixture of substituted 2-amino 1,3,4-triazoles of the formula

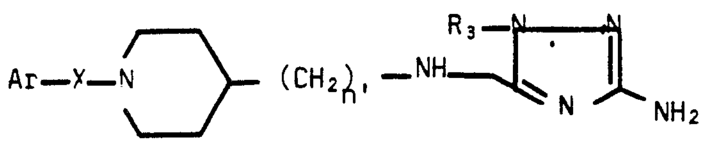

and

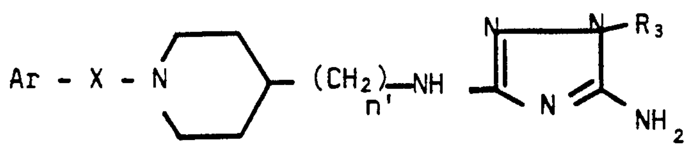

which are separed by means of physical methods.

14. A process according to claim 13 which includes the further step of salification by adding a mineral or organic acid.

15. A process according to claim 13 or claim 14 which includes the further step of resolution by means of salification using an optically - active acid.